# EUROPEAN PATENT SPECIFICATION

(11) **EP 1 530 466 B1**
(45) Date of publication and mention of the grant of the patent: **10.12.2014**
(21) Application number: 03792292.9
(22) Date of filing: 11.08.2003
(51) Int. Cl.: A61K 31/496, A61P 11/00, A61K 45/06

(54) **Use of Nintedanib for the treatment of lung fibrosis**
Verwendung von Nintedanib zur Behandung der Lungenfibrose
Utilisation du nintedanibe dans le traitement de la fibrose pulmonaire

(30) Priority: 16.08.2002 DE 10237423
(43) Date of publication of application: 18.05.2005
(60) Divisional application: 10184940.4
(73) Proprietor: Boehringer Ingelheim Pharma GmbH & Co. KG, 55216 Ingelheim am Rhein (DE)
(72) Inventor: ROTH, Gerald, Jürgen, 88400 Biberach (DE); HAUPTMANN, Rudolf, A-2483 Ebreichsdorf (AT); HILBERG, Frank, A-1050 Wien (AT); COLBATZKY, Florian, 88441 Stafflangen (DE); ERNST, Steffen, S-43537 Mölnlycke (SE); HECKEL, Armin, 88400 Biberach an der Riss 1 (DE); STEFANIC, Martin, Friedrich, 88447 Warthausen-Birkenhard (DE); WALTER, Rainer, 88400 Biberac am Riss (DE)
(86) International application number: PCT/EP2003/008890
(87) International publication number: WO 2004/017948

(56) References cited:
- WO-A-00/18734
- WO-A-00/73297
- WO-A-01/25238
- WO-A-01/27080
- WO-A-01/27081
- WO-A-01/40215
- WO-A-01/72711
- WO-A-02/36564
- WO-A-97/40019
- WO-A-98/11095
- WO-A-98/54156
- WO-A-99/52869
- WO-A2-98/24432
- JP-A- H0 892 248
- Toby Maher ET AL: "Reducing lung function decline in patients with idiopathic pulmonary fibrosis: potential of nintedanib", Drug Design, Development and Therapy, 1 June 2013 (2013-06-01), page 503, XP055125113, ISSN: 1177-8881, DOI: 10.2147/DDDT.S38833

## Description

### TECHNICAL FIELD OF THE INVENTION

This invention relates to the use of the compound (AK) for the manufacture of a pharmaceutical composition for the treatment of immunologic diseases or pathological conditions involving an immunologic component.

### BACKGROUND OF THE INVENTION

Compound (AK), its preparation as well as the pharmacological activity of this compound based on inhibition of kinases, e.g. VEGFR-2, suitable for therapy in oncology, is disclosed in WO 01/27081.

Lck, a further tyrosine kinase belonging to the src family of tyrosine kinases not mentioned in the reference cited above, is functionally required for T-cell activation through the T-cell antigen receptor (TCR) (see A.E. Nel: T-cell activation through antigen receptor. Part 1: Signaling components, signaling pathways, and signal integration at the T-cell antigen receptor synapse. J. Allergy Clin Immunol, 109, 5, 758-770, 2002) and possibly T-cell survival (Seddon, B.; Legname, G.; Tomlinson, P.; Zamoyska, R. : Long-term survival but impaired homeostatic proliferation of naive T cells in the absence of p56(Ick). Science 290: 127-131, 2000). Therefore a Lck inhibitor has a high possible therapeutic potential in the treatment of T-cell mediated diseases, e.g. in the treatment of immunologic diseases. Certain autoimmune diseases such as inflammatory diseases (for example inflammatory bowel disease, rheumatoid arthritis, glomerulonephritis and lung fibrosis, psoriasis, hypersensitivity reactions of the skin, atherosclerosis, restenosis, allergic asthma, multiple sclerosis and type 1 diabetes are believed to be associated with inappropriate T cell activation (J. H. Hanke et al., Inflamm. Res., 1995, 357). In addition the acute rejection of transplanted organs as well as Graft versus Host Disease (GvHD) after allogeneic bone marrow and stem cell transplantation can also be interpreted as consequence of inappropriate T cell activation. Lck inhibitors offer an approach for treatment of the indications mentioned hereinbefore. Agents of this kind would offer therapy for transplant rejection and autoimmune diseases whilst avoiding toxicities associated with the commonly used, less selective immunosuppressants. The leading agent for prevention or treatment of transplant rejection is cyclosporin A which, although effective, is often associated with side-effects such as renal damage and hypertension which results in kidney failure in a substantial number of patients. It is contemporary practice to treat rheumatoid arthritis initially with symptom relief agents such as NSAIDs, which have no effect on disease progression and are often associated with unwanted side-effects. A rationally based, disease modifying agent, without such deleterious side-effects, would therefore offer significant benefits in the prevention or treatment of transplant rejection or autoimmune conditions such as rheumatoid arthritis.

There is considerable evidence that VEGF plays a key role in the pathogegenesis in rheumatoid arthritis, especially in the formation of the pannus (Paleolog EM, Arthritis Res 2002;4 Suppl 3:S81-90, Pavonen et al, J Rheumatol 2002 Jan;29(1):39-45, Afuwape AO et al, Histol Histopathol 2002;17(3):961-72). Thus, combined inhibition of VEGFR-tyrosine kinases and Lck is considered of potentially high benefit for patients with this disease. The same considerations can be applied to psoriasis and inflammatory bowel disease (Folkman J, Nat Med. 1995 Jan;1(1):27-31. Review; Griga T et al, Hepatogastroenterology 2002 Jan-Feb;49(43):116-23, Creamer D et al, Arch Dermatol 2002 Jun;138(6):791-6).

### BRIEF SUMMARY OF THE INVENTION

In view of the work cited above there is a clear need for compounds being active as Lck inhibitors in order to treat T-cell mediated diseases, e.g. in the treatment of lung fibrosis, an immunologic disease or pathological condition involving an immunologic component. It is an object of the invention to provide compound (AK), or a tautomer, a stereoisomer or a physiologically acceptable salt thereof, for use in the treatment of lung fibrosis. A second object of the invention is a pharmaceutical composition comprising compound (AK) together with one or more other drugs selected from nonsteroidal anti-inflammatory drugs (NSAIDs), steroids, disease-modifying antirheumatic drugs (DMARDs), immunsuppressives and biologic response modifiers for use in the treatment of lung fibrosis.

### DETAILED DESCRIPTION OF THE INVENTION

It has now surprisingly been found that the compound
(AK) (Z)-3-(1-(4-(N-((4-methyl-piperazin-1-yl)-methylcarbonyl)-N-methyl-amino)-phenylamino)-1-phenyl-methylene]-6-methoxycarbonyl-2-indolinone;
a tautomer, a stereoisomer or a physiologically acceptable salt thereof, is an effective inhibitor of Lck and therefore is especially suitable and effective in the treatment of lung fibrosis. Compound (AK) is described in WO 01/27081.

Compound (AK) can be administered orally, parenterally or rectally. Oral administration is preferred.

In oral, rectal or topical administration the compound may be given, if required in divided doses, in a daily dosage of 0.1 to 20 mg/kg body weight, preferably 0.5 to 20 mg/kg body weight, most preferred 1 to 10 mg/kg body weight.

Parenterally the compound may be administered in lower doses, for instance in a total daily dosage of 0.01 to 5 mg/kg body weight, preferably 0.05 to 2 mg/kg body weight, most preferred 0.1 to 1 mg/kg body weight.

For administration the compound may be formulated with one or more conventional inert carriers and/or diluents as known in the art, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, stearyl alcohol, carboxymethylcellulose or fatty substances such as hard fat or suitable mixtures thereof in conventional galenic preparations such as plain or coated tablets, lozenges, hard or soft capsules, dispersible powders or granules, syrups or elixirs, injectable solutions, ampoules, aqueous or oily suspensions, emulsions, solutions, sprays, creams, ointments, gels, or suppositories. Suitable galenic formulations are disclosed in the document cited hereinbefore.

Furthermore, the compound (AK) may be administered in combination, simultaneously or sequentially, with one or more other drugs selected from NSAIDs, steroids, DMARDs, immunsuppressives, biologic response modifiers and bronchodilators, as defined below.

Suitable NSAIDS are selected from the non-selective COX-inhibitors acetylsalicyclic acid, mesalazin, ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen,
indomethacin, sulindac, tolmetin, zomepirac, nabumetone, diclofenac, fenclofenac, alclofenac, bromfenac, ibufenac, aceclofenac, acemetacin, fentiazac, clidanac, etodolac, oxpinac,
mefenamic acid, meclofenamic acid, flufenamic acid, nifluminic acid, tolfenamic acid, diflunisal, flufenisal, piroxicam, tenoxicam, lornoxicam and nimesulide and the pharmaceutically acceptable salts thereof, the selective COX 2-inhibitors meloxicam, celecoxib and rofecoxib and the pharmaceutically acceptable salts thereof.

Suitable steroids are prednisone, prednisolone, methylprednisolone, dexamethasone, budenoside, fluocortolone and triamcinolone.

Suitable DMARDs are sulfasalazine, olsalazine, chloroquin, gold derivatives (Auranofin), D-penicillamine and cytostatics such as methotrexate and cyclophosphamide.

Suitable immunsuppressives are cyclosporine A and derivatives thereof, mycophenolatemofetil, FK 506, OKT-3, ATG, 15-desoxyspergualin, mizoribine, misoprostol, rapamycin, reflunomide and azathioprine.

Suitable biologic response modifiers are interferon beta, anti-TNF-alpha (Etanercept), IL-10, anti-CD3 or anti-CD25.

Suitable bronchodilators are ipratropiumbromide, oxytropiumbromide, tiotropiumbromide, epinephrinehydrochloride, salbutamole, terbutalinsulfate, fenoterolhydrobromide, salmeterole and formoterole.

In such combinations each active ingredient can be administered either in accordance with its usual dosage range or a dose below its usual dosage range. The dosage for the combined NSAIDs, steroids, DMARDs, immunsuppressives and biologic response modifiers is appropriately 1/50 of the lowest dose normally recommended up to 1/1 of the normally recommended dosage, preferably 1/20 to 1/2 and more preferably 1/10 to 1/5. The normally recommended dose for the combined drug should be understood to be the dose disclosed for example in Rote Liste® 2002, Editio Cantor Verlag Aulendorf, Germany, or in Physician's Desk Reference.

It can be expected that combination treatment comprising administration of Lck inhibitors together with a second drug selected from those mentioned hereinbefore may provide synergistic efficacy thus allowing significant dose reduction compared to the normally needed dose to produce a therapeutic effect. This would be especially beneficial with regard to medications having a high risk of adverse side-effects as is the case with non-selective COX inhibitors, cyclosporin A or DMARDs.

Viewed from a second aspect the present invention also relates to a pharmaceutical composition comprising
(a) compound (AK) or a tautomer, a stereoisomer or a physiologically acceptable salt thereof,
(b) and one or more other drugs selected from NSAIDs, steroids, DMARDs, immunsuppressives and biologic response modifiers, as defined before, optionally together with pharmaceutically acceptable diluents and/or carriers, as a combined preparation or a kit of parts containing components (a) and (b) in separate containments for simultaneous, separate or sequential use in treatment of lung fibrosis.

### Example 1: Non-radioactive kinase assay (Ick)

### Methodology

The Ick enzyme comprises the entire Ick molecule except the first nine amino acids which are replaced by an His-tag for purification purposes. The enzyme is affinity purified.

The assay mix is assembled in a well of a 96-well round bottom microtiter plate and contains 10 µl PBS (either as such or with an inhibitor dissolved at an appropriate concentration), 20 µl substrate solution (200 mM Hepes, pH=7,4; 50 mM MgAc₂; 1 mM Na₃VO₄; 250 µg/ml poly-Glu-Tyr (Sigma P0275); 200 ng/ml biotinylated peptide (biot-Ala-Glu-Glu-Glu-Ile-Tyr-Gly-Glu-Phe-Glu-Ala-Lys-Lys-Lys-Lys) and 20 µl of 2,5 ng/µl enzyme (diluted from affinity purified stock with enzyme dilution buffer, EDB: 20 mM Hepes, pH=7,4, 130 mM NaCl, 0,05% Triton X-100).

The reaction is started by the addition of 50 µl 500 µM ATP (in 10 mM MgAc₂) and is performed at room temperature. After 30 minutes 50 µl stop solution (20 mM Hepes, pH=7,4;250 mM EDTA) are added and 100 µl of this solution transfered to the well of a streptavidin coated microtiter plate (SA-MTP, Boehringer Mannheim, #1664-760). The solution is incubated for one hour at room temparature and the supernatant discarded. The well is washed twice with 300 µl PBS.

The streptavidin bound biotinylated peptide is incubated for 1 hour at room temperature with 100 µl Eu³⁺-labelled anti-phosphotyrosine antibody solution (0,3 mg/ml DELFIA-Eu-labelled PT66 (Wallac, AD0041); 50 mM Tris, pH=7,8; 0,05% Tween 20; 0,5% (w7v) BSA (Serva, diagnostic grade) under gentle agitation. The well is washed three times with 1x Delfia wash buffer (Wallac, 1244-114, 25x concentrate, diluted with water) and finally 100 µl Delfia enhancement solution (Wallac, 1244-105) are added.

Time resolved fluorescense is measured in a Wallac Victor2 1420 Multilabel Counter, excitation is at 340 nm, emission is measured at 615 nm (delay time 400 µsec, window time 1000 µspec).

### Results

In two independent experiments the IC₅₀s of compound (AK) on the kinase has been determined. The data (mean values) obtained is summarised in the following table:

| compound | Lck; IC₅₀ *[nM]* |
|---|---|
| (AK) | 16 |

## Claims

1. Compound
(AK) (Z)-3-(1-(4-(N-((4-methyl-piperazin-1-yl)-methylcarbonyl)-N-methyl-amino)-phenylamino)-1-phenyl-methylene]-6-methoxycarbonyl-2-indolinone, or
a tautomer, a stereoisomer or a physiologically acceptable salt thereof, for use in the treatment of lung fibrosis.

2. Compound (AK), or a tautomer, a stereoisomer or a physiologically acceptable salt thereof, for use in the treatment of lung fibrosis according to claim 1, wherein the treatment is optionally in combination with one or more other drugs selected from
the NSAIDs
acetylsalicyclic acid, mesalazin,
ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen,
indomethacin, sulindac, tolmetin, zomepirac, nabumetone, diclofenac, fenclofenac, alclofenac, bromfenac, ibufenac, aceclofenac, acemetacin, fentiazac, clidanac, etodolac, oxpinac,
mefenamic acid, meclofenamic acid, flufenamic acid, nifluminic acid, tolfenamic acid, diflunisal, flufenisal, piroxicam, tenoxicam, lornoxicam, nimesulide,
meloxicam, celecoxib and rofecoxib,
and the pharmaceutically acceptable salts thereof,
the steroids
prednisone, prednisolone, methylprednisolone, dexamethasone, budenoside, fluocortolone and triamcinolone,
the DMARDs
sulfasalazine, olsalazine, chloroquin, Auranofin, D-penicillamine, methotrexate and cyclophosphamide,
the immunosuppressives
cyclosporin A, mycophenolatemofetil, FK 506, OKT-3, ATG, 15-desoxyspergualin, mizoribine, misoprostol, rapamycin, reflunomide, azathioprine,
the biologic response modifiers
interferon beta, anti-TNF-alpha (Etanercept), IL-10, anti-CD3 and anti-CD25,
and the bronchodilators
ipratropiumbromide, oxytropiumbromide, tiotropiumbromide, epinephrinehydrochloride, salbutamole, terbutalinsulfate, fenoterolhydrobromide, salmeterole and formoterole.

3. Compound (AK), or a tautomer, a stereoisomer or a physiologically acceptable salt thereof, for use in the treatment of lung fibrosis according to claim 1 or 2, wherein the compound (AK) is administered orally, parenterally or rectally.

4. Compound (AK), or a tautomer, a stereoisomer or a physiologically acceptable salt thereof, for use in the treatment of lung fibrosis according to claim 3, wherein the compound is given in oral or rectal administration in a daily dosage of 0.1 to 20 mg/kg body weight and in parenteral administration in a total daily dosage of 0.01 to 5 mg/kg body weight.

5. Pharmaceutical composition comprising
(a) Compound
(AK) (Z)-3-(1-(4-(N-((4-methyl-piperazin-1-yl)-methylcarbonyl)-N-methyl-amino)-phenylamino)-1-phenyl-methylene]-6-methoxycarbonyl-2-indolinone, or
a tautomer, a stereoisomer or a physiologically acceptable salt thereof, and
(b) One or more other drugs selected from selected from
the NSAIDs
acetylsalicyclic acid, mesalazin,
ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen,
indomethacin, sulindac, tolmetin, zomepirac, nabumetone, diclofenac, fenclofenac, alclofenac, bromfenac, ibufenac, aceclofenac, acemetacin, fentiazac, clidanac, etodolac, oxpinac,
mefenamic acid, meclofenamic acid, flufenamic acid, nifluminic acid, tolfenamic acid, diflunisal, flufenisal, piroxicam, tenoxicam, lornoxicam, nimesulide,
meloxicam, celecoxib and rofecoxib,
and the pharmaceutically acceptable salts thereof,
the steroids
prednisone, prednisolone, methylprednisolone, dexamethasone, budenoside, fluocortolone and triamcinolone,
the DMARDs
sulfasalazine, olsalazine, chloroquin, Auranofin, D-penicillamine, methotrexate and cyclophosphamide,
the immunosuppressives
cyclosporin A, mycophenolatemofetil, FK 506, OKT-3, ATG, 15-desoxyspergualin, mizoribine, misoprostol, rapamycin, reflunomide and azathioprine,
the biologic response modifiers
interferon beta, anti-TNF-alpha (Etanercept), IL-10, anti-CD3 and anti-CD25,
and the bronchodilators
ipratropiumbromide, oxytropiumbromide, tiotropiumbromide, epinephrinehydrochloride, salbutamole, terbutalinsulfate, fenoterolhydrobromide, salmeterole and formoterole,
optionally together with pharmaceutically acceptable diluents and/or carriers, as a combined preparation or a kit of parts containing components (a) and (b) in separate containments for simultaneous, separate or sequential use in treatment of lung fibrosis.

6. The composition for the use of claim 5, wherein component (b) is an NSAID selected from
acetylsalicyclic acid, mesalazin,
ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen,
indomethacin, sulindac, tolmetin, zomepirac, nabumetone, diclofenac, fenclofenac, alclofenac, bromfenac, ibufenac, aceclofenac, acemetacin, fentiazac, clidanac, etodolac, oxpinac,
mefenamic acid, meclofenamic acid, flufenamic acid, nifluminic acid, tolfenamic acid, diflunisal, flufenisal, piroxicam, tenoxicam, lornoxicam, nimesulide,
meloxicam, celecoxib and rofecoxib,
and the pharmaceutically acceptable salts thereof.

7. The composition for the use of claim 5, wherein component (b) is a steroid selected from
prednisone, prednisolone, methylprednisolone, dexamethasone, budenoside, fluocortolone and triamcinolone.

8. The composition for the use of claim 5, wherein component (b) is a DMARD selected from sulfasalazine, olsalazine, chloroquin, Auranofin, D-penicillamine, methotrexate and cyclophosphamide.

9. The composition for the use of claim 5, wherein component (b) is an immunosuppressive selected from cyclosporin A, mycophenolatemofetil, FK 506, OKT-3, ATG, 15-desoxyspergualin, mizoribine, misoprostol, rapamycin, reflunomide and azathioprine.

10. The composition for the use of claim 5, wherein component (b) is a biologic response modifier selected from interferon beta, anti-TNF-alpha (Etanercept), IL-10, anti-CD3 and anti-CD25.

11. The composition for the use of claim 5, wherein component (b) is a bronchodilator selected from ipratropiumbromide, oxytropiumbromide, tiotropiumbromide, epinephrinehydrochloride, salbutamole, terbutalinsulfate, fenoterolhydrobromide, salmeterole and formoterole.

## Patentansprüche

1. Verbindung
(AK) (Z)-3-(1-(4-(N-((4-Methyl-piperazin-1-yl)-methylcarbonyl)-N-methyl-amino)-phenyl-amino)-1-phenyl-methylen]-6-methoxycarbonyl-2-indolinon oder
ein Tautomer, ein Stereoisomer oder ein physiologisch akzeptables bzw. annehmbares Salz hiervon zur Verwendung in der Behandlung von Lungenfibrose.

2. Verbindung (AK) oder ein Tautomer, ein Stereoisomer oder ein physiologisch akzeptables bzw. annehmbares Salz hiervon zur Verwendung in der Behandlung von Lungenfibrose nach Anspruch 1, wobei die Behandlung gegebenenfalls in Kombination mit ein oder mehreren anderen Wirkstoffen durchgeführt wird, ausgewählt aus
den NSAIDs
Acetylsalicyclsäure, Mesalazin,
Ibuprofen, Naproxen, Flurbiprofen, Fenoprofen, Fenbufen, Ketoprofen, Indoprofen, Pirprofen, Carprofen, Oxaprozin, Pranoprofen, Miroprofen, Tioxaprofen, Suprofen, Alminoprofen, Tiaprofensäure, Fluprofen,
Indomethacin, Sulindac, Tolmetin, Zomepirac, Nabumeton, Diclofenac, Fenclofenac, Alclofenac, Bromfenac, Ibufenac, Aceclofenac, Acemetacin, Fentiazac, Clidanac, Etodolac, Oxpinac,
Mefenaminsäure, Meclofenaminsäure, Flufenaminsäure, Nifluminsäure, Tolfenaminsäure, Diflunisal, Flufenisal, Piroxicam, Tenoxicam, Lornoxicam, Nimesulid,
Meloxicam, Celecoxib und Rofecoxib,
und die pharmazeutisch akzeptablen bzw. annehmbaren Salze hiervon,
den Steroiden
Prednison, Prednisolon, Methylprednisolon, Dexamethason, Budenosid, Fluocortolon und Triamcinolon,
den DMARDs
Sulfasalazin, Olsalazin, Chloroquin, Auranofin, D-Penicillamin, Methotrexat und Cyclophosphamid,
den Immunsupressiva
Cyclosporin A, Mycophenolat-Mofetil, FK 506, OKT-3, ATG, 15-Desoxyspergualin, Mizoribin, Misoprostol, Rapamycin, Reflunomid, Azathioprin,
den biologischen Reaktionsmodifikatoren
Interferon beta, Anti-TNF-alpha (Etanercept), IL-10, Anti-CD3 und Anti-CD25,
und den Bronchodilatoren
Ipratropiumbromid, Oxytropiumbromid, Tiotropiumbromid, Epinephrinhydrochlorid, Salbutamol, Terbutalinsulfat, Fenoterolhydrobromid, Salmeterol und Formoterol.

3. Verbindung (AK) oder ein Tautomer, ein Stereoisomer oder ein physiologisch akzeptables bzw. annehmbares Salz hiervon zur Verwendung in der Behandlung von Lungenfibrose nach Anspruch 1 oder 2, wobei die Verbindung (AK) oral, parenteral oder rektal verabreicht wird.

4. Verbindung (AK) oder ein Tautomer, ein Stereoisomer oder ein physiologisch akzeptables bzw. annehmbares Salz hiervon, zur Verwendung in der Behandlung von Lungenfibrose nach Anspruch 3, wobei die Verbindung durch orale oder rektale Verabreichung in einer täglichen Dosierung von 0,1 bis 20 mg/kg Körpergewicht und durch parenterale Verabreichung in einer täglichen Gesamtdosis von 0,01 bis 5 mg/kg Köpergewicht gegeben wird.

5. Pharmazeutische Zusammensetzung, umfassend
(a) Verbindung
(AK) (Z)-3-(1-(4-(N-((4-Methyl-piperazin-1-yl)-methylcarbonyl)-N-methyl-amino)-phenylamino)-1-phenyl-methylen]-6-methoxycarbonyl-2-indolinon oder
ein Tautomer, ein Stereoisomer oder ein physiologisch akzeptables bzw. annehmbares Salz hiervon, und
(b) ein oder mehrere andere Arzneistoffe, ausgewählt aus
den NSAIDs
Acetylsalicyclsäure, Mesalazin,
Ibuprofen, Naproxen, Flurbiprofen, Fenoprofen, Fenbufen, Ketoprofen, Indoprofen, Pirprofen, Carprofen, Oxaprozin, Pranoprofen, Miroprofen, Tioxaprofen, Suprofen, Alminoprofen, Tiaprofensäure, Fluprofen,
Indomethacin, Sulindac, Tolmetin, Zomepirac, Nabumeton, Diclofenac, Fenclofenac, Alclofenac, Bromfenac, Ibufenac, Aceclofenac, Acemetacin, Fentiazac, Clidanac, Etodolac, Oxpinac,
Mefenaminsäure, Meclofenaminsäure, Flufenaminsäure, Nifluminsäure, Tolfenaminsäure, Diflunisal, Flufenisal, Piroxicam, Tenoxicam, Lornoxicam, Nimesulid,
Meloxicam, Celecoxib und Rofecoxib,
und die pharmazeutisch akzeptablen bzw. annehmbaren Salze hiervon,
den Steroiden
Prednison, Prednisolon, Methylprednisolon, Dexamethason, Budenosid, Fluocortolon und Triamcinolon,
den DMARDs
Sulfasalazin, Olsalazin, Chloroquin, Auranofin, D-Penicillamin, Methotrexat und Cyclophosphamid,
den Immunsupressiva
Cyclosporin A, Mycophenolat-Mofetil, FK 506, OKT-3, ATG, 15-Desoxyspergualin, Mizoribin, Misoprostol, Rapamycin, Reflunomid, Azathioprin,
den biologischen Reaktionsmodifikatoren
Interferon beta, Anti-TNF-alpha (Etanercept), IL-10, Anti-CD3 und Anti-CD25,
und den Bronchodilatoren
Ipratropiumbromid, Oxytropiumbromid, Tiotropiumbromid, Epinephrinhydrochlorid, Salbutamol, Terbutalinsulfat, Fenoterolhydrobromid, Salmeterol und Formoterol,
gegebenenfalls zusammen mit pharmazeutisch akzeptablen bzw. annehmbaren Verdünnungsmitteln und/oder Trägern, als eine kombinierte Zubereitung oder ein Kit-of-parts, enthaltend die Komponenten (a) und (b) in getrennten Behältnissen zur gleichzeitigen, getrennten oder aufeinanderfolgenden Verwendung in der Behandlung von Lungenfibrose.

6. Zusammensetzung zur Verwendung nach Anspruch 5, wobei die Komponente (b) ein NSAID darstellt, ausgewählt aus
Acetylsalicyclsäure, Mesalazin,
Ibuprofen, Naproxen, Flurbiprofen, Fenoprofen, Fenbufen, Ketoprofen, Indoprofen, Pirprofen, Carprofen, Oxaprozin, Pranoprofen, Miroprofen, Tioxaprofen, Suprofen, Alminoprofen, Tiaprofensäure, Fluprofen,
Indomethacin, Sulindac, Tolmetin, Zomepirac, Nabumeton, Diclofenac, Fenclofenac, Alclofenac, Bromfenac, Ibufenac, Aceclofenac, Acemetacin, Fentiazac, Clidanac, Etodolac, Oxpinac,
Mefenaminsäure, Meclofenaminsäure, Flufenaminsäure, Nifluminsäure, Tolfenaminsäure, Diflunisal, Flufenisal, Piroxicam, Tenoxicam, Lornoxicam, Nimesulid,
Meloxicam, Celecoxib und Rofecoxib,
und die pharmazeutisch akzeptablen bzw. annehmbaren Salze hiervon.

7. Zusammensetzung zur Verwendung nach Anspruch 5, wobei die Komponente (b) ein Steroid darstellt, ausgewählt aus
Prednison, Prednisolon, Methylprednisolon, Dexamethason, Budenosid, Fluocortolon und Triamcinolon.

8. Zusammensetzung zur Verwendung nach Anspruch 5, wobei die Komponente (b) ein DMARD darstellt, ausgewählt aus
Sulfasalazin, Olsalazin, Chloroquin, Auranofin, D-Penicillamin, Methotrexat und Cyclophosphamid.

9. Zusammensetzung zur Verwendung nach Anspruch 5, wobei die Komponente (b) ein Immunosuppressivum darstellt, ausgewählt aus
Cyclosporin A, Mycophenolat-Mofetil, FK 506, OKT-3, ATG, 15-Desoxyspergualin, Mizoribin, Misoprostol, Rapamycin, Reflunomid, Azathioprin.

10. Zusammensetzung zur Verwendung nach Anspruch 5, wobei die Komponente (b) einen biologischen Reaktionsmodifikator darstellt, ausgewählt aus
Interferon beta, Anti-TNF-alpha (Etanercept), IL-10, Anti-CD3 und Anti-CD25.

11. Zusammensetzung zur Verwendung nach Anspruch 5, wobei die Komponente (b) einen Bronchodilator darstellt, ausgewählt aus
Ipratropiumbromid, Oxytropiumbromid, Tiotropiumbromid, Epinephrinhydrochlorid, Salbutamol, Terbutalinsulfat, Fenoterolhydrobromid, Salmeterol und Formoterol.

## Revendications

1. Composé
(AK) (Z)-3-(1-(4-(N-((4-méthyl-pipérazin-1-yl)-méthylcarbonyl)-N-méthyl-amino)-phénylamino)-1-phényl-méthylène]-6-méthoxycarbonyl-2-indolinone, ou
un tautomère, un stéréoisomère ou un sel physiologiquement acceptable de celui-ci, pour son utilisation dans le traitement de la fibrose pulmonaire.

2. Composé (AK), ou un tautomère, un stéréoisomère ou un sel physiologiquement acceptable de celui-ci, pour son utilisation dans le traitement de la fibrose pulmonaire selon la revendication 1, dans lequel le traitement est réalisé optionellement en combinaison avec un ou plusieurs autres médicaments sélectionnés parmi
les AINS
acide acétylsalicylique, mésalazine, ibuprofène, naproxène, flurbiproféne, fénoprofène, fenbufène, cétoprofène, indoprofène, pirprofène, carprofène, oxaprozine, pranoprofène, miroprofène, tioxaprofène, suprofène, alminoprofène, acide tiaprofénique, fluprofène,
indométhacine, sulindac, tolmétine, zomépirac, nabumétone, diclofénac, fenclofénac, alclofénac, bromfénac, ibufénac, acéclofénac, acémétacine, fentiazac, clidanac, étodolac, oxpinac,
acide méfénamique, acide méclofénamique, acide flufénamique, acide nifluminique, acide tolfénamique, diflunisal, flufénisal, piroxicam, ténoxicam, lornoxicam, nimésulide,
méloxicam, célécoxib et rofécoxib,
et leurs sels pharmaceutiques acceptables,
les stéroïdes
prednisone, prednisolone, méthylprednisolone, dexaméthasone, budénoside, fluocortolone et triamcinolone,
les ARMM
sulfasalazine, olsalazine, chloroquine, auranofine, D-pénicillamine, méthotrexate et cyclophosphamide, les immunosuppresseurs
cyclosporine A, mycophénolatémofétil, FK 506, OKT-3, ATG, 15-désoxyspergualine, mizoribine, misoprostol, rapamycine, réflunomide et azathioprine,
les modificateurs de réponse biologique
interféron bêta, anti-TNF-alpha (Etanercept), IL-10, anti-CD3 et anti-CD25,
et les bronchodilatateurs
bromure d'ipratropium, bromure d'oxytropium, bromure de tiotropium, chlorhydrate d'épinéphrine, salbutamole, sulfate de terbutaline, bromhydrate de fénotérol, salmétérole et formotérole.

3. Composé (AK), ou un tautomère, un stéréoisomère ou un sel physiologiquement acceptable de celui-ci, pour son utilisation dans le traitement de la fibrose pulmonaire selon la revendication 1 ou 2, dans lequel le composé (AK) est administré par voie orale, parentérale ou rectale.

4. Composé (AK), ou un tautomère, un stéréoisomère ou un sel physiologiquement acceptable de celui-ci, pour son utilisation dans le traitement de la fibrose pulmonaire selon la revendication 3, dans lequel le composé est administré par voie orale ou rectale à une dose quotidienne de 0,1 à 20 mg/kg de poids corporel et par voie parentérale à une dose quotidienne totale de 0,01 à 5 mg/kg de poids corporel.

5. Composition pharmaceutique comprenant
(a) un composé
(AK) (Z)-3-(1-(4-(N-((4-méthyl-pipérazin-1-yl)-méthylcarbonyl)-N-méthyl-amino)-phénylamino)-1-phényl-méthylène]-6-méthoxycarbonyl-2-indolinone, ou
un tautomère, un stéréoisomère ou un sel physiologiquement acceptable de celui-ci, et
(b) un ou plusieurs autres médicaments sélectionnés parmi
les AINS
acide acétylsalicylique, mésalazine,
ibuprofène, naproxène, flurbiproféne, fénoprofène, fenbufène, cétoprofène, indoprofène, pirprofène, carprofène, oxaprozine, pranoprofène, miroprofène, tioxaprofène, suprofène, alminoprofène, acide tiaprofénique, fluprofène,
indométhacine, sulindac, tolmétine, zomépirac, nabumétone, diclofénac, fenclofénac, alclofénac, bromfénac, ibufénac, acéclofénac, acémétacine, fentiazac, clidanac, étodolac, oxpinac,
acide méfénamique, acide méclofénamique, acide flufénamique, acide nifluminique, acide tolfénamique, diflunisal, flufénisal, piroxicam, ténoxicam, lornoxicam, nimésulide,
méloxicam, célécoxib et rofécoxib,
et leurs sels pharmaceutiques acceptables,
les stéroïdes
prednisone, prednisolone, méthylprednisolone, dexaméthasone, budénoside, fluocortolone et triamcinolone,
les ARMM
sulfasalazine, olsalazine, chloroquine, auranofine, D-pénicillamine, méthotrexate et cyclophosphamide,
les immunosuppresseurs
cyclosporine A, mycophénolatémofétil, FK 506, OKT-3, ATG, 15-désoxyspergualine, mizoribine, misoprostol, rapamycine, réflunomide et azathioprine,
les modificateurs de réponse biologique
interféron bêta, anti-TNF-alpha (Etanercept), IL-10, anti-CD3 et anti-CD25,
et les bronchodilatateurs
bromure d'ipratropium, bromure d'oxytropium, bromure de tiotropium, chlorhydrate d'épinéphrine, salbutamole, sulfate de terbutaline, bromhydrate de fénotérol, salmétérole et formotérole,
optionellement avec des diluants et/ou supports pharmaceutiquement acceptables, sous la forme d'une préparation combinée ou d'un kit de parties contenant les composants (a) et (b) dans des compartiments séparés pour une utilisation simultanée, séparée ou séquentielle dans le traitement de la fibrose pulmonaire.

6. Composition pour l'utilisation selon la revendication 5, dans laquelle le composant (b) est un AINS sélectionné parmi
l'acide acétylsalicylique, la mésalazine,
l'ibuprofène, le naproxène, le flurbiproféne, le fénoprofène, le fenbufène, le cétoprofène, l'indoprofène, le pirprofène, le carprofène, l'oxaprozine, le pranoprofène, le miroprofène, le tioxaprofène, le suprofène, l'alminoprofène, l'acide tiaprofénique, le fluprofène,
l'indométhacine, le sulindac, la tolmétine, le zomépirac, la nabumétone, le diclofénac, le fenclofénac, l'alclofénac, le bromfénac, l'ibufénac, l'acéclofénac, l'acémétacine, le fentiazac, le clidanac, l'étodolac, l'oxpinac,
l'acide méfénamique, l'acide méclofénamique, l'acide flufénamique, l'acide nifluminique, l'acide tolfénamique, le diflunisal, le flufénisal, le piroxicam, le ténoxicam, le lornoxicam, le nimésulide,
le méloxicam, le célécoxib et le rofécoxib,
et leurs sels pharmaceutiques acceptables.

7. Composition pour l'utilisation selon la revendication 5, dans laquelle le composant (b) est un stéroïde sélectionné parmi
la prednisone, la prednisolone, la méthylprednisolone, la dexaméthasone, le budénoside, la fluocortolone et la triamcinolone.

8. Composition pour l'utilisation selon la revendication 5, dans laquelle le composant (b) est un ARMM sélectionné parmi
la sulfasalazine, l'olsalazine, la chloroquine, l'auranofine, la D-pénicillamine, le méthotrexate et le cyclophosphamide.

9. Composition pour l'utilisation selon la revendication 5, dans laquelle le composant (b) est un immunosuppresseur sélectionné parmi la cyclosporine A, le mycophénolatémofétil, le FK 506, l'OKT-3, l'ATG, la 15-désoxyspergualine, la mizoribine, le misoprostol, la rapamycine, le réflunomide et l'azathioprine.

10. Composition pour l'utilisation selon la revendication 5, dans laquelle le composant (b) est un modificateur de réponse biologique sélectionné parmi l'interféron bêta, un anti-TNF-alpha (Etanercept), l'IL-10, un anti-CD3 et un anti-CD25.

11. Composition pour l'utilisation selon la revendication 5, dans laquelle le composant (b) est un bronchodilatateur sélectionné parmi le bromure d'ipratropium, le bromure d'oxytropium, le bromure de tiotropium, le chlorhydrate d'épinéphrine, le salbutamole, le sulfate de terbutaline, le bromhydrate de fénotérol, le salmétérole et le formotérole.
